# EUROPÄISCHE PATENTSCHRIFT

(11) **EP 3 551 126 B1**
(45) Veröffentlichungstag und Bekanntmachung des Hinweises auf die Patenterteilung: **15.07.2020**
(21) Anmeldenummer: 17818077.4
(22) Anmeldetag: 06.12.2017
(51) Int. Cl.: A61C 19/045, G01R 33/24, A61B 6/14, A61B 8/13

(54) **VERFAHREN ZUR ERFASSUNG DER BEWEGUNG EINES KIEFERGELENKS**
METHOD FOR DETECTING THE MOVEMENT OF A TEMPOROMANDIBULAR JOINT
PROCÉDÉ DE DÉTECTION DU MOUVEMENT D'UNE ARTICULATION DE MÂCHOIRE

(30) Priorität: 06.12.2016 DE 102016224182
(43) Veröffentlichungstag der Anmeldung: 16.10.2019
(73) Patentinhaber: Sirona Dental Systems GmbH, 64625 Bensheim (DE)
(72) Erfinder: RASCHE, Volker, 89155 Erbach (DE); WUNDRAK, Stefan, 64625 Bensheim (DE)
(74) Vertreter: Özer, Alpdeniz
(86) Internationale Anmeldenummer: PCT/EP2017/081641
(87) Internationale Veröffentlichungsnummer: WO 2018/104372

(56) Entgegenhaltungen:
- WO-A1-2009/118391
- DE-A1-102009 027 356
- DE-B3-102014 209 437
- SHUO ZHANG ET AL: "Real-Time Magnetic Resonance Imaging of Temporomandibular Joint Dynamics", THE OPEN MEDICAL IMAGING JOURNAL, BENTHAM OPEN, NL, Bd. 5, 1. Januar 2011 (2011-01-01), Seiten 1-7, XP002742190, ISSN: 1874-3471, DOI: 10.2174/1874347101105010001

## Beschreibung

### Technisches Gebiet

Die Erfindung betrifft ein Verfahren zur Erfassung und Darstellung der Bewegung eines einen Unterkiefer und einen Oberkiefer verbindenden Kiefergelenks eines Patienten mittels Magnetresonanztomographie.

### Stand der Technik

Typische Verfahren zur Erfassung und Darstellung der Bewegung eines Kiefergelenks, wie sie beispielsweise zur Diagnose von Kraniomandibulären Dysfunktionen eingesetzt wird, kombinieren Bewegungsdaten aus einem Axiographiescan mit den Bilddaten eines DVT-Scans. Häufig wird die so gewonnene Darstellung noch durch die Daten eines Oberflächenscans ergänzt.

Nachteilig ist jedoch, sowohl die Strahlenbelastung durch einen DVT-Scan, das Unvermögen des DVT-Scans Weichgewebe darzustellen sowie der hohe zeitliche Aufwand.

Aus "Prospective Real Time Head Motion Correction Using Inductively Coupled Wireless NMR Probes", S.Sengupta et al., Magn. Reson. Med. (2014) 72(4), 971-985, ist ein Verfahren zum Tracken einer Kopfbewegung eines Patienten während einer MRT-Aufnahme des Kopfes bekannt, wobei induktiv koppelnde Markerspulen eingesetzt werden.

Vor diesem Hintergrund besteht die Aufgabe der Erfindung darin, den Stand der Technik weiterzubilden und ein Verfahren zur einfachen und schnellen Erfassung und Darstellung der Bewegung eines Kiefergelenks unter Einsatz von magnetresonanztomographischer Vermessung bereitzustellen.

Aus der DE 10 2014 209 437 B3 ist ein Verfahren zur Erzeugung magnetresonanztomographischer Aufnahmen von zyklischer Bewegung bekannt, bei dem die Kaubewegung eines Kiefergelenks beispielhaft erläutert wird. Dabei wird eine Zuordnung einzelner Zwischenpositionen rechnerisch ermittelt.

Aus der DE 10 2009 027 356 A1 ist es bekannt, die relative Position von Oberkiefer und Unterkiefer zueinander über einen Öffnungswinkel zu bestimmen.

### Darstellung der Erfindung

Ein Gegenstand der Erfindung ist ein Verfahren zur Erfassung und Darstellung der Bewegung eines einen Unterkiefer und einen Oberkiefer verbindenden Kiefergelenks eines Patienten mittels Magnetresonanztomographie, wobei mindestens ein magnetresonanztomographisch-sichtbarer Marker mittels eines Befestigungsmittels an dem Unterkiefer des Patienten befestigt wird.

Während eines ersten Messzeitintervalls werden zeitlich nacheinander mehrere magnetresonanztomographische Messdatensätze eines Aufnahmevolumens erzeugt, wobei zumindest das Kiefergelenk und/oder mindestens ein Teil des Unterkiefers sowie der mindestens eine Marker während des ersten Messzeitintervalls in dem Aufnahmevolumen positioniert sind, der Unterkiefer während des ersten Messzeitintervalls relativ zu dem Oberkiefer eine Bewegung ausführt und die Bewegung des Unterkiefers eine ersten Position relativ zu dem Oberkiefer umfasst.

In jedem Messdatensatz wird eine mindestens zweidimensionale Markerposition des mindestens einen Markers in dem Aufnahmevolumen ermittelt und anhand der bestimmten Markerpositionen eine Marker-Bewegungskurve erstellt, wobei ein der erste Position des Unterkiefers zu dem Oberkiefer entsprechender Punkt auf der Marker-Bewegungskurve ermittelt wird.

Während eines zweiten Messzeitintervalls wird mindestens ein Bilddatensatz des Aufnahmevolumens erzeugt, wobei zumindest das Kiefergelenk und/oder mindestens ein Teil des Unterkiefers sowie der mindestens eine Marker während des zweiten Messzeitintervalls in dem Aufnahmevolumen positioniert sind und sich der Unterkiefer während des zweiten Messzeitintervalls relativ zu dem Oberkiefer in der ersten Position befindet.

Aus dem Bilddatensatz wird ein erstes Modell und ein zweites Modell ermittelt, wobei das erste Modell zumindest einen Teil des Oberkiefers und/oder einen die Kiefergelenkpfanne, auch Fossa mandibularis genannt, umfassenden Teil des Schläfenbeins darstellt und das zweite Modell zumindest einen Teil des Unterkiefers darstellt. Aus dem Bilddatensatz wird ferner eine relative Position des Markers zu dem ersten Modell und zu dem zweiten Modell bestimmt.

Anhand der Marker-Bewegungskurve und der relativen Position des ersten Modells und des zweiten Modells zu dem Marker wird ein Bewegungsverlauf des zweiten Modells relativ zu dem ersten Modell berechnet und dargestellt.

Es versteht sich, dass der Verfahrensschritt des Marker-Positionierens vor dem ersten und dem zweiten Messzeitintervall bzw. dem Erzeugen der ersten Messdatensätze und des Bilddatensatzes ausgeführt wird.

Es versteht sich weiterhin, dass das erste Messzeitintervall zeitlich vor oder auch nach dem zweiten Messzeitintervall liegen kann.

Die Bewegung des Kiefergelenks ist eine Kaubewegung oder ein periodisches Öffnen und Schließen des Mundes oder eine beliebige andere während des ersten Messzeitintervall ausgeführte Kieferbewegung.

Die erste Position des Unterkiefers relativ zu dem Oberkiefer ist beispielsweise der natürliche Schlussbiss oder eine durch einen Zahnabdruck kodierte Position. Die erste Position ist sowohl in der Bewegungskurve ermittelbar als auch für die Erzeugung des Bilddatensatzes zuverlässig reproduzierbar.

Entsprechend erhält man aus dem Bilddatensatz, der bei unbewegten Kiefer in der ersten Position erzeugt wird, die relative Position des ersten Modells und des zweiten Modells zu dem Marker in eben der ersten Position, z.B dem Schlussbiss. Es versteht sich, dass die relative Position des einen Modells zu dem Marker auch bestimmt ist, wenn die relative Position des einen Modells zu dem anderen Modell und die relativen Position des anderen Modells zu dem Marker ermittelt ist. Anhand der relativen Position des Markers zu dem zweiten und dem zweiten Modell in der erste Position und dem der ersten Position entsprechenden Punkt in der Marker-Bewegungskurve, kann die Bewegung aus der Marker-Bewegungskurve in eine Bewegung des zweiten Modells, z.B. dem Unterkiefer, relativ zu dem ersten Modell, z.B. dem Oberkiefer, übertragen werden.

Der magnetresonanztomographisch-sichtbare Marker umfasst jeden Marker, der geeignet ist, innerhalb magnetresonanztomographisch erfasster Daten eindeutig erkannt zu werden.

Die während des ersten Messzeitintervalls erzeugten Messdatensätze dienen jeweils lediglich der Bestimmung der mindestens zweidimensionalen, bevorzugt dreidimensionalen Position des Markers im Aufnahmevolumen zum Zeitpunkt der Erzeugung des jeweiligen Messdatensatzes. Die Messdatensätze dienen nicht der bildlichen Erfassung des Aufnahmevolumens bzw. von Unterkiefer, Oberkiefer und/oder Marker.

Entsprechend handelt es sich bei den Messdatensätzen um beliebige mittels Magnetresonanztomographie erzeugbare Messdatensätze, die Informationen über die Markerposition liefern. Hierfür reichen beispielsweise einzelne magnetresonanztomographische Projektionsaufnahmen aus, wobei als Projektionsaufnahme eine im Rahmen einer Messsequenz entlang einer k-Raum Trajektorie erfasste Datenmenge bezeichnet wird. Die zur Bestimmung einer Markerposition abgetasteten Trajektorien sind bevorzugt orthogonal zueinander ausgerichtet. Die Messdatensätze entsprechen beispielsweise jeweils einer radialen Abtastung entlang eines Radialstrahls bzw. einer sogenannten Spoke innerhalb des Aufnahmevolumens bzw. den bei der Abtastung erfassten Daten, wobei eine solche Abtastung typischerweise nur ca. 1 bis 5 ms in Anspruch nimmt.

Dadurch ist die für das Erzeugen eines Messdatensatzes benötigte Zeit sehr kurz, insbesondere im Vergleich zu der für die Erzeugung eines Bilddatensatzes notwendigen Zeit, was es ermöglicht, viele Messdatensätze in kurzer zeitlicher Folge aufzunehmen und so die Bewegung des am Unterkiefer angebrachten Markers, also unterschiedliche Markerpositionen mit einer hohen zeitlichen Auflösung zu erfassen. Die jeweils aus den einzelnen Messdatensätzen ermittelten Markerpositionen werden zu einer Marker-Bewegungskurve zusammengesetzt. Es versteht sich, dass hierfür alle oder zumindest alle geeigneten Markerpositionen oder eine geeignete Auswahl an Markerpositionen verwendet werden.

Während des ersten Messzeitintervalls werden Informationen über die Bewegung des Kiefergelenks unabhängig von einer bildlichen Darstellung bzw. von einer Erfassung von Bildinformationen ermittelt

Eine bildliche Darstellung zumindest eines Teils des Kieferbereichs wird während des zweiten Messzeitintervalls als Bilddatensatz erfasst.Der Bilddatensatz kann gemäß jedes bekannten bildgebender Verfahren, z.B. mittels magnetresonanztomographischen Verfahrens erfasst werden, welches dazu geeignet ist zumindest Teile des Kiefers und/oder des Kiefergelenks jeweils mit Zähnen und Zahnfleisch und/oder Knochen sowie den Marker in mindestens zwei, bevorzugt in drei Dimensionen zu erfassen.

Aus dem Bilddatensatz werden, beispielsweise durch Segmentieren, ein erstes Modell und ein zweites Modell extrahiert. Das erste Modell umfasst den Oberkiefer oder einen Teil des Oberkiefers und/oder einen Teil des Schläfenbeins, z.B. die gelenkbildende Gelenkpfanne, auch Unterkiefergrube oder Fossa mandibularis genannt. Das zweite Modell umfasst den Unterkiefer oder ein Teil des Unterkiefers, z.B. den gelenkbildenden walzenförmigen Gelenkkopf des Unterkiefers (Caput mandibulae). Das erste und das zweite Modell sind mindestens zweidimensional, bevorzugt dreidimensional.

Die Kiefergelenkbewegung wird anschließend durch eine Bewegung des zweiten Modells relativ zu dem ersten Modell bildlich dargestellt, wobei Bewegungsdaten aus der Bewegungskurve entnommen werden.

Für die Darstellung der Kieferbewegung wird somit eine statische, bildliche Darstellung des Kiefers, nämlich der Bilddatensatz, in einen oberen und einen unteren Bereich, z.B. Oberkiefer und Unterkiefer zerlegt und anschließend der untere Bereich gegenüber dem oberen Bereich entsprechend der ermittelten Bewegungskurve des Markers bewegt.

Es versteht sich, dass die Darstellung in Form eines Videos, durch überlagerte Bilder oder in beliebiger anderer Form vorgenommen werden kann.

Ein Vorteil der erfindungsgemäßen Erfassung von Kiefergelenkbewegungen ist, dass Bewegungsdaten und Bilddaten in getrennten Verfahrensschritten erfasst werden. Hierdurch können die Verfahrensschritte jeweils auf die unterschiedlichen Anforderungen angepasst werden. Es können insbesondere zeitlich und/oder hinsichtlich der Datenqualität und/oder der Strahlenbelastung optimierte Verfahren gewählt werden.

Vorteilhafterweise umfasst jeder Messdatensatz mindestens drei 1D-Projektionsaufnahmen oder mindestens zwei 2D-Projektionsaufnahmen. So ist es auf einfache und schnelle Weise möglich, anhand von lediglich zwei bzw. drei Projektionsaufnahmen die Markerposition, z.B. als Lösung eines entsprechend aufgestellten Gleichungssystems, zu bestimmen.

Gemäß einer alternativen Ausführungsform umfasst jeder erste Messdatensatz genau eine 1D-Projektionen, wobei jeweils eine Markerposition anhand von mindestens zwei Messdatensätzen ermittelt wird.

In vorteilhaften Ausführungsformen wird der mindestens eine Bilddatensatz magnetresonanztomographisch, radiographisch oder auch optisch erzeugt, wobei bei radiographischer Erzeugung der mindestens eine Marker zumindest in einem hinsichtlich seiner Position bekannten Bereich röntgenopak und bei einer optischen Erzeugung optisch eindeutig identifizierbar ist.

Wird der mindestens eine Bilddatensatz mit der für die Erzeugung der Messdatensätze verwendeten magnetresonanztomographischen Aufnahmevorrichtung erzeugt, so erübrigt sich eine weitere Vorrichtung für die Erzeugung des Bilddatensatzes. Hierdurch besteht ferner die Möglichkeit, ein neuerliches Positionieren eines Patienten für die Erzeugung des Bilddatensatzes einzusparen. Ferner stellt die magnetresonanztomographische Erzeugung der Bilddaten eine gute Darstellung von Weichgeweben und eine gute Erkennbarkeit des Diskus Articularis sicher. Außerdem ist es bei Verwendung derselben Aufnahmevorrichtung möglich, auch für den Bilddatensatz eine dreidimensionale Position des Markers im Aufnahmevolumen zu bestimmen und für die Übertragung der Marker-Bewegungskurve auf die Bewegung des Unterkiefers zu verwenden.

Für einen magnetresonanztomographischen Bilddatensatz, also eine bildliche Darstellung bzw. ein zweidimensionales oder dreidimensionales statisches Modell zumindest eines Teils eines Kieferbereichs sind typischerweise eine Vielzahl von Projektionsaufnahmen bzw. k-Raum Profilen nötigt, beispielsweise 200 x 200, aus denen der Bilddatensatz erzeugt wird.

Der Bilddatensatz kann auch durch eine optische Vermessung des Unterkiefers, des Oberkiefer und des mindestens einen positionierten Markers erzeugt werden, z.B. mittels einer intraoralen Kamera. Auch eine Kombination von magnetresonanztomographischen und optischen Messergebnissen für den Bilddatensatz ist möglich. Optische Messdaten ermöglichen eine sehr hohe räumliche Auflösung der Zahnoberfläche sowie weitergehende Therapieformen, wie die Fertigung von Zahnschienen.

Ebenso ist es möglich, den Bilddatensatz vollständig oder teilweise aus radiographischen Messdaten zu erzeugen.

Vorteilhafterweise ist der mindestens eine Marker ein magnetresonanztomographisch aktiver oder ein magnetresonanztomographisch semi-aktiver Marker, besonders vorteilhaft eine Spule oder eine Kombination aus einer Spule und einem in der Spule angeordneten und mit einer Flüssigkeit gefüllten Körper, wobei die Flüssigkeit bevorzugt mit einem Kontrastmittel dotiert ist.

Ein semi-aktiver Marker stellt eine induktiv an das Empfangssystem gekoppelte Vorrichtung dar, beispielsweise einen Schwingkreis mit einer Mikrospule und einem innerhalb der Mikrospule angeordneten mit einer dotierten Flüssigkeit gefüllten Körper, während ein aktiver Marker eine mit einem eigenen Empfangskanal verbundene Empfangsspule aufweist. Der semi-aktive Marker liefert aufgrund der Erhöhung des effektiven Flusses und Flipwinkels innerhalb der Mikrospule ein besonders deutliches Signal, welches mittels einer Empfangsspule des MRT-Systems detektierbar ist. Mittels des aktiven Markers wird die Positionsinformation unabhängig von der Empfangsspule des MRT-Systems aufgezeichnet.

Das Erzeugen eines Messdatensatzes zur Positionsbestimmung eines semi-aktiven Markers umfasst beispielsweise eine Messsequenz mit einem Flipwinkel von wenigen Grad, wobei der semi-aktive Marker das B1+Feld lokal verstärkt, also den Flipwinkel lokal erhöht. Hierdurch liefert der semi-aktive Marker im Vergleich zu dem restlichen Field-of-View ein deutlich verstärktes Signal, so dass eine Positionsbestimmung einfach und sehr zuverlässig möglich ist.

Wird ein magnetresonanztomographisch aktiver Marker verwendet, werden die Messdatensätze direkt mittels des aktiven Markers erfasst, während der Bilddatensatz im Falle einer magnetresonanztomographischen Erzeugung mittels einer Empfangsspule der verwendeten magnetresonanztomographischen Vorrichtung ermittelt wird.

Mittels der dotierten Flüssigkeit im Spulenzentrum wird die Stärke des messbaren Signals verstärkt. Eine genaue Positionsbestimmung bzw. ein möglichst präzises Bewegungstracking ist beispielsweise durch Bestimmen der jeweiligen Position des Schwerpunkts des Körpers in den Messdatensätzen möglich.

Vorteilhafterweise weist der mindestens eine Marker eine dreidimensional eindeutige Struktur auf, z.B. ein Kreuz, um eine schnelle und einfache Bestimmung der dreidimensionalen Position des Markers zu ermöglichen.

Vorteilhafterweise wird der mindestens eine Marker außerhalb der Mundhöhle des Patienten positioniert. Gemäß einer alternativen Ausführungsform wird der mindestens eine Marker innerhalb einer Mundhöhle des Patienten an einer Fläche mindestens eines Zahns, z.B. an einer lingualen Fläche eines oder mehrerer Schneidezähne oder an einer oralen Fläche eines oder mehrerer Molaren, positioniert.

Vorteilhafterweise ist das Befestigungsmittel ein Paraokklusal-Löffel, wobei der Paraokklusal-Löffel an den Zähnen des Unterkiefers angeordnet und mit diesen lösbar verbunden wird, wobei der mindestens eine Marker an mindestens einem aus der Mundhöhle herausragenden Arm des Paraokklusal-Löffels angeordnet wird und wobei mindestens eine Empfangsspule zur Erzeugung der Messdatensätze und/oder des Bilddatensatzes in einem Bereich der Kiefergelenke angeordnet wird.

Ein Paraokklusal-Löffel ist eine bogenförmig ausgebildete, meist individualisierte Vorrichtung, die beispielsweise mittels eines Klebers an den Zähnen des Oberkiefers oder des Unterkiefers befestigt werden kann, wodurch der Paraokklusal-Löffel der Bewegung des jeweiligen Kiefers folgt. Vorliegend ist der Paraokklusal-Löffel aus einem Material, z.B. Kunststoff, welches die magnetresonanztomographischen Aufnahmen möglichst wenig beeinträchtigt, beispielsweise, indem es möglichst geringe Signale produziert.

### Kurzbeschreibung der Zeichnungen

Ausführungsbeispiele der Erfindung sind in der Zeichnung dargestellt. Es zeigt die
- Fig. 1: Verfahrensschritte gemäß einer ersten Ausführungsform des erfindungsgemäßen Verfahrens,
- Fig. 2: eine schematische Darstellung eines relativ zu einem Oberkiefermodell bewegten Unterkiefermodells.

### Ausführungsbeispiele

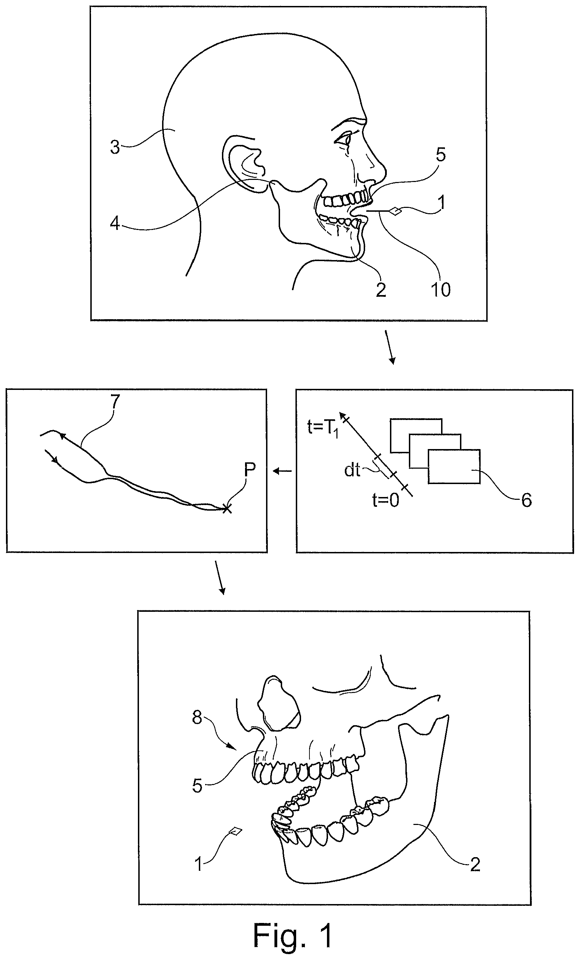

In Figur 1 sind Verfahrensschritte des erfindungsgemäßen Verfahrens gemäß einer ersten Ausführungsform skizziert. Zuerst wird ein Marker 1 mittels eines Befestigungsmittels 10 an einem Unterkiefer 2 eines Patienten 3 befestigt. Der Marker 1 besteht aus einem magnetresonanztomographisch sichtbaren Material. Das Befestigungsmittel 10 ist im dargestellten Ausführungsbeispiel ein mittels eines Klebemittels an Zähnen des Unterkiefers 2 befestigter Paraokklusal-Löffel.

Anschließend wird mittels einer magnetresonanztomographischen Aufnahmevorrichtung (nicht dargestellt) eine durch eine Bewegung des Kiefergelenks 4 hervorgerufene Bewegung des Markers 1 vermessen, wozu Unterkiefer 2, Oberkiefer 5 und Marker 1 in einem Aufnahmevolumen der Aufnahmevorrichtung positioniert und das Aufnahmevolumen vermessen wird. Hierzu werden während eines ersten Messzeitintervalls T1 in kurzen zeitlichen Abständen dt mehrere Messdatensätze 6 mittels der MR-Aufnahmevorrichtung erzeugt. Anhand jedes Messdatensatzes 6 wird anschließend eine dreidimensionale Position des Markers 1 zu dem Zeitpunkt der Erstellung des jeweiligen Messdatensatzes 6 ermittelt. Die ermittelten Markerpositionen 1 in Abhängigkeit von der Zeit t werden als Marker-Bewegungskurve 7 abgespeichert. Auf der Marker-Bewegungskurve 7 wird ein Punkt P ermittelt, wobei der Punkt P der ersten Position P1 des Unterkiefers relativ zu dem Oberkiefer, z.B. dem natürlichen Schlussbiss entspricht.

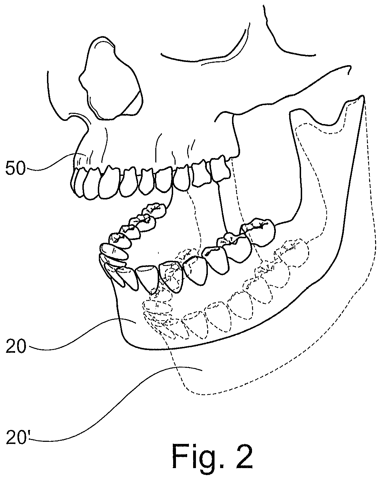

Anschließend wird eine bildliche Aufnahme des unbewegten Kiefers erstellt. Hierzu wird gemäß dem dargestellten Ausführungsbeispiel mittels der MR-Aufnahmevorrichtung während eines zweiten Messzeitintervalls T2 ein Bilddatensatz 8 erzeugt, wobei Unterkiefer, Oberkiefer und Marker im Aufnahmevolumen der Aufnahmevorrichtung verbleiben und der Unterkiefer sich relativ zu dem Oberkiefer in der ersten Position P1, z. B. dem natürlichen Schlussbiss oder einer durch einen im Mund positionierten Zahnabdruck/Aufbiss definierten Position, befindet. Der Bilddatensatz 8 umfasst im dargestellten Ausführungsbeispiel dreidimensionale Bildinformationen des Unterkiefers 2, des Oberkiefers 5 und des Markers 1. Anhand des Bilddatensatzes 8 wird einerseits durch Segmentieren ein dreidimensionales Unterkiefermodell als zweites Modell 20 und ein dreidimensionales Oberkiefermodell 50 als erstes Modell 50 erzeugt, die in Figur 2 dargestellt sind. Andererseits wird anhand des Bilddatensatzes eine relative Position des Markers 1 zu dem Unterkiefermodell 20 und dem Oberkiefermodell 50 ermittelt. Gemäß dem dargestellten Ausführungsbeispiel wird ferner eine dreidimensionale Position des Markers 1 im Aufnahmevolumen bestimmt.

Anhand der dreidimensionalen Position des Markers 1 im Aufnahmevolumen und der relativen Position des Markers 1 zu dem Unterkiefermodell 20 und dem Oberkiefermodell 50 wird aus der Marker-Bewegungskurve 7 ein Bewegungsverlauf des Unterkiefermodells 20, 20' relativ zu dem Oberkiefermodell 50 berechnet und, wie in Figur 2 skizziert, dargestellt.

## Patentansprüche

1. Verfahren zur Erfassung und Darstellung der Bewegung eines einen Unterkiefer (2) und einen Oberkiefer (5) verbindenden Kiefergelenks (4) eines Patienten (3) mittels Magnetresonanztomographie, wobei
- mindestens ein magnetresonanztomographisch-sichtbarer Marker (1) mittels eines Befestigungsmittels (10) an dem Unterkiefer (2) des Patienten (3) befestigt wird,
- während eines ersten Messzeitintervalls (T1) zeitlich nacheinander mehrere magnetresonanztomographische Messdatensätze (6) eines Aufnahmevolumens erzeugt werden,
- zumindest das Kiefergelenk (4) und/oder mindestens ein Teil des Unterkiefers (2) sowie der mindestens eine Marker (1) während des ersten Messzeitintervalls (T1) in dem Aufnahmevolumen positioniert sind,
- der Unterkiefer (2) während des ersten Messzeitintervalls (T1) relativ zu dem Oberkiefer (5) eine Bewegung ausführt,
- die Bewegung des Unterkiefers (2) eine erste Position (P1) relativ zu dem Oberkiefer (5) umfasst,
- in jedem Messdatensatz (6) eine mindestens zweidimensionale Markerposition des mindestens einen Markers (1) in dem Aufnahmevolumen ermittelt wird,
- anhand der bestimmten Markerpositionen eine Marker-Bewegungskurve (7) erstellt wird,
- ein der ersten Position (P1) des Unterkiefers (2) relativ zu dem Oberkiefer (5) entsprechender Punkt auf der Marker-Bewegungskurve (7) ermittelt wird oder bekannt ist,
- während eines zweiten Messzeitintervalls (T2) mindestens ein Bilddatensatz (8) des Aufnahmevolumens erzeugt wird,
- zumindest das Kiefergelenk (4) und/oder mindestens ein Teil des Unterkiefers (2) sowie der mindestens eine Marker (1) während des zweiten Messzeitintervalls (T2) in dem Aufnahmevolumen positioniert sind,
- sich der Unterkiefer (2) während des zweiten Messzeitintervalls (T2) in der ersten Position (P1)relativ zu dem Oberkiefer (5) befindet,
- aus dem Bilddatensatz (8) ein zumindest einen Teil des Oberkiefers (5) und/oder einen die Kiefergelenkpfanne (Fossa mandibularis) umfassenden Teil des Schläfenbeins darstellendes erstes Modell (50) und ein zumindest einen Teil des Unterkiefers (2) darstellendes zweites Modell (20) ermittelt wird,
- aus dem Bilddatensatz (8) eine relative Position des mindestens einen Markers (1) zu dem ersten Modell (50) und zu dem zweiten Modell (20) bestimmt wird und
- ein Bewegungsverlauf des zweiten Modells (20, 20') relativ zu dem ersten Modell (50) anhand der Marker-Bewegungskurve (7) und der relativen Position des ersten Modells (50) und des zweiten Modells (20) zu dem mindestens einen Marker (1) berechnet und dargestellt wird.

2. Verfahren nach Anspruch 1, **dadurch gekennzeichnet, dass** jeder Messdatensatz (6) mindestens drei 1D-Projektionsaufnahmen oder mindestens zwei 2D-Projektionsaufnahmen umfasst.

3. Verfahren nach Anspruch 1, **dadurch gekennzeichnet, dass** jeder Messdatensatz (6) genau eine 1D-Projektsionsaufnahme umfasst, wobei jeweils eine Markerposition anhand von mindestens zwei Messdatensätzen (6) ermittelt wird.

4. Verfahren nach einem der Ansprüche 1 bis 3, **dadurch gekennzeichnet, dass** der Bilddatensatz (8) magnetresonanztomographisch erzeugt wird.

5. Verfahren nach einem der Ansprüche 1 bis 4, **dadurch gekennzeichnet, dass** der Bilddatensatz (8) optisch erzeugt wird, wobei der mindestens eine Marker (1) optisch eindeutig identifizierbar ist.

6. Verfahren nach einem der Ansprüche 1 bis 5, **dadurch gekennzeichnet, dass** der Bilddatensatz (8) radiographisch erzeugt wird, wobei der mindestens eine Marker (1) zumindest in einem bekannten Bereich röntgenopak ist.

7. Verfahren nach einem der Ansprüche 1 bis 6, **dadurch gekennzeichnet, dass** der mindestens eine Marker (1) ein magnetresonanztomographisch aktiver Marker (1) oder ein magnetresonanztomographisch semi-aktiver Marker (1) ist.

8. Verfahren nach Anspruch 7, **dadurch gekennzeichnet, dass** der aktive oder semi-aktive Marker (1) eine Spule umfasst.

9. Verfahren nach Anspruch 8, **dadurch gekennzeichnet, dass** in der Spule ein mit einer Flüssigkeit gefüllter Körper angeordnet ist.

10. Verfahren nach Anspruch 9, **dadurch gekennzeichnet, dass** die Flüssigkeit mit einem Kontrastmittel dotiert ist.

11. Verfahren nach einem der Ansprüche 1 bis 10, **dadurch gekennzeichnet, dass** der mindestens eine Marker (1) eine dreidimensional eindeutige Struktur aufweist.

12. Verfahren nach einem der Ansprüche 1 bis 11, **dadurch gekennzeichnet, dass** der mindestens eine Marker (1) innerhalb einer Mundhöhle des Patienten (3) an einer Fläche mindestens eines Zahns oder außerhalb der Mundhöhle des Patienten (3) angeordnet wird.

13. Verfahren nach einem der Ansprüche 1 bis 12, **dadurch gekennzeichnet, dass** das Befestigungsmittel (10) ein Paraokklusal-Löffel ist, wobei der Paraokklusal-Löffel an den Zähnen des Unterkiefers (2) angeordnet und mit diesen lösbar verbunden wird, wobei der mindestens eine Marker (1) an mindestens einem aus einer Mundhöhle des Patienten (3) herausragenden Arm des Paraokklusal-Löffels angeordnet wird und mindestens eine Empfangsspule zur Erzeugung der ersten Messdatensätze (6) und/oder des Bilddatensatzes (8) in einem Bereich um die Kiefergelenke (4) angeordnet wird.

## Claims

1. A method for detecting and displaying the movement of a temporomandibular joint (4) of a patient (3), which joint connects a lower jaw (2) and an upper jaw (5), by means of magnetic resonance tomography, wherein
- at least one marker (1) that is visible using magnetic resonance tomography is fastened to the lower jaw (2) of the patient (3) by way of a fastening means (10),
- a plurality of magnetic resonance tomography measurement data sets (6) of a recording volume is generated in chronological succession during a first measurement time interval (T1),
- at least the temporomandibular joint (4) and/or at least one part of the lower jaw (2) and the at least one marker (1) are positioned in the recording volume during the first measurement time interval (T1),
- the lower jaw (2) performs a movement relative to the upper jaw (5) during the first measurement time interval (T1),
- the movement of the lower jaw (2) has a first position (P1) relative to the upper jaw (5),
- one at least two-dimensional marker position of the at least one marker (1) in the recording volume is determined in each measurement data set (6),
- a marker movement curve (7) is created on the basis of the determined marker positions,
- a point on the marker movement curve (7) corresponding to the first position (P1) of the lower jaw (2) relative to the upper jaw (5) is determined or is known,
- at least one image data set (8) of the recording volume is generated during a second measurement time interval (T2),
- at least the temporomandibular joint (4) and/or at least one part of the lower jaw (2) and the at least one marker (1) are positioned in the recording volume during the second measurement time interval (T2),
- the lower jaw (2) is in the first position (P1) relative to the upper jaw (5) during the second measurement time interval (T2),
- a first model (50) representing at least one part of the upper jaw (5) and/or a part of the temporal bone comprising the temporomandibular socket (Fossa mandibularis) and a second model (20) representing at least one part of the lower jaw (2) are determined from the image data set (8),
- a position of the at least one marker (1) relative to the first model (50) and the second model (20) is determined from the image data set (8) and
- a movement pattern of the second model (20, 20') relative to the first model (50) is calculated and displayed based on the marker movement curve (7) and the relative position of the first model (50) and the second model (20) to the at least one marker (1).

2. The method according to claim 1, **characterised in that** each measurement data set (6) comprises at least three 1D projection images or at least two 2D projection images.

3. The method according to claim 1, **characterised in that** each measurement data set (6) comprises exactly one 1D projection image, wherein a marker position is determined in each case on the basis of at least two measurement data sets (6).

4. The method according to any one of claims 1 to 3, **characterised in that** the image data set (8) is generated by means of magnetic resonance tomography.

5. The method according to any one of claims 1 to 4, **characterised in that** the image data set (8) is generated optically, wherein the at least one marker (1) is optically uniquely identifiable.

6. The method according to any one of claims 1 to 5, **characterised in that** the image data set (8) is generated radiographically, wherein the at least one marker (1) is radiopaque at least in a known region.

7. The method according to any one of claims 1 to 6, **characterised in that** the at least one marker (1) is a magnetic resonance tomographically active marker (1) or a magnetic resonance tomographically semi-active marker (1).

8. The method according to claim 7, **characterised in that** the active or semi-active marker (1) comprises a coil.

9. The method according to claim 8, **characterised in that** a body filled with a liquid is arranged in the coil.

10. The method according to claim 9, **characterised in that** the liquid is doped with a contrast agent.

11. The method according to any one of claims 1 to 10, **characterised in that** the at least one marker (1) has a three-dimensionally unique structure.

12. The method according to any one of claims 1 to 11, **characterised in that** the at least one marker (1) is arranged inside an oral cavity of the patient (3) on a surface of at least one tooth, or outside the oral cavity of the patient (3).

13. The method according to any one of claims 1 to 12, **characterised in that** the fastening means (10) is a paraocclusal spoon, wherein the paraocclusal spoon is arranged on the teeth of the lower jaw (2) and is detachably connected thereto, wherein the at least one marker (1) is arranged on at least one arm of the para-occlusal spoon projecting from an oral cavity of the patient (3) and at least one receiving coil for generating the first measurement data sets (6) and/or the image data set (8) is arranged in a region around the temporomandibular joints (4).

## Revendications

1. Procédé de détection et de représentation du mouvement d'une articulation temporo-mandibulaire (4) d'un patient (3) reliant une maxillaire inférieure (2) et une maxillaire supérieure (5) au moyen d'une imagerie par résonance magnétique, dans lequel
- au moins un marqueur visible par imagerie par résonance magnétique (1) est fixé à la maxillaire inférieure (2) du patient (3) à l'aide d'un moyen de fixation (10),
- au cours d'un premier intervalle de temps de mesure (T1), plusieurs ensembles de données de mesure par imagerie par résonance magnétique (6) d'un volume de clichés sont générés les uns après les autres,
- au moins l'articulation temporo-mandibulaire (4) et/ou au moins une partie de la maxillaire inférieure (2) ainsi que ledit au moins un marqueur (1) sont positionnés dans le volume de clichés pendant le premier intervalle de temps de mesure (T1),
- la maxillaire inférieure (2) effectuant un mouvement par rapport à la maxillaire supérieure (5) pendant le premier intervalle de temps de mesure (T1),
- le mouvement de la maxillaire inférieure (2) comprenant une première position (P1) par rapport à la maxillaire supérieure (5),
- dans chaque ensemble de données de mesure (6), une position de marqueur au moins bidimensionnelle dudit au moins un marqueur (1) étant déterminée dans le volume de clichés,
- une courbe de déplacement (7) du marqueur étant créée sur la base des positions de marqueur déterminées,
- un point sur la courbe de déplacement (7) du marqueur correspondant à la première position (P1) de la maxillaire inférieure (2) par rapport à la maxillaire supérieure (5) étant déterminé ou connu,
- au moins un ensemble de données d'image (8) du volume de clichés étant généré pendant un second intervalle de temps de mesure (T2),
- au moins l'articulation temporo-mandibulaire (4) et/ou au moins une partie de la maxillaire inférieure (2) et ledit au moins un marqueur (1) étant positionnés dans le volume de clichés pendant le second intervalle de temps de mesure (T2),
- la maxillaire inférieure (2) se trouvant dans la première position (P1) par rapport à la maxillaire supérieure (5) pendant le second intervalle de temps de mesure (T2),
- à partir de l'ensemble de données d'images (8), il est déterminé un premier modèle (50) représentant au moins une partie de la maxillaire supérieure (5) et/ou une partie de l'os temporal comprenant la fosse mandibulaire (Fossa mandibularis) et un second modèle (20) représentant au moins une partie de la maxillaire inférieure (2),
- il est déterminé à partir de l'ensemble de données d'image (8) une position relative dudit au moins un marqueur (1) par rapport au premier modèle (50) et au second modèle (20) et
- il est calculé et représenté un tracé de mouvement du second modèle (20, 20') par rapport au premier modèle (50) sur la base de la courbe de mouvement (7) du marqueur et de la position relative du premier modèle (50) et du second modèle (20) par rapport au moins un marqueur (1).

2. Procédé selon la revendication 1, **caractérisé en ce que** chaque ensemble de données de mesure (6) comprend au moins trois clichés de projection 1D ou au moins deux clichés de projection 2D.

3. Procédé selon la revendication 1, **caractérisé en ce que** chaque ensemble de données de mesure (6) comprend exactement un cliché de projection 1D, respectivement une position du marqueur étant déterminée sur la base d'au moins deux ensemble de données de mesure (6).

4. Procédé selon l'une quelconque des revendications 1 à 3, **caractérisé en ce que** l'ensemble de données d'image (8) est généré par imagerie par résonance magnétique.

5. Procédé selon l'une quelconque des revendications 1 à 4, **caractérisé en ce que** l'ensemble de données d'image (8) est généré optiquement, ledit au moins un marqueur (1) étant optiquement identifiable de manière unique.

6. Procédé selon l'une quelconque des revendications 1 à 5, **caractérisé en ce que** l'ensemble de données d'image (8) est généré par radiographie, ledit au moins un marqueur (1) étant radio-opaque au moins dans une zone connue.

7. Procédé selon l'une quelconque des revendications 1 à 6, **caractérisé en ce que** ledit au moins un marqueur (1) est un marqueur actif (1) d'imagerie à résonance magnétique ou un marqueur semi-actif (1) d'imagerie à résonance magnétique.

8. Procédé selon la revendication 7, **caractérisé en ce que** le marqueur actif ou semi-actif (1) comprend une bobine.

9. Procédé selon la revendication 8, **caractérisé en ce qu'**un corps rempli de liquide est disposé dans la bobine.

10. Procédé selon la revendication 9, **caractérisé en ce que** le liquide est dopé avec un agent de contraste.

11. Procédé selon l'une quelconque des revendications 1 à 10, **caractérisé en ce que** ledit au moins un marqueur (1) a une structure tridimensionnelle unique.

12. Procédé selon l'une quelconque des revendications 1 à 11, **caractérisé en ce que** ledit au moins un marqueur (1) est disposé à l'intérieur d'une cavité buccale du patient (3) sur une surface d'au moins une dent ou à l'extérieur de la cavité buccale du patient (3).

13. Procédé selon l'une quelconque des revendications 1 à 12, **caractérisé en ce que** le moyen de fixation (10) est une cuillère para-occlusale, la cuillère para-occlusale étant disposée sur les dents de la maxillaire inférieure (2) et étant reliée de manière détachable à celle-ci, ledit au moins un marqueur (1) étant disposé sur au moins un bras de la cuillère para-occlusale faisant saillie de la cavité buccale du patient (3) et au moins une bobine réceptrice pour générer les premiers ensembles de données de mesure (6) et/ou l'ensemble de données d'image (8) étant disposée dans une zone autour des articulations temporo-mandibulaires (4).
